(19) Europäisches Patentamt
European Patent Office
Office européen des brevets

(11) Publication number: **0 320 898 A2**

(12) # EUROPEAN PATENT APPLICATION

(21) Application number: **88120880.5**

(51) Int. Cl.4: **C07B 57/00 , C07C 87/28**

(22) Date of filing: **14.12.88**

(30) Priority: **18.12.87 US 134674**

(43) Date of publication of application: **21.06.89 Bulletin 89/25**

(84) Designated Contracting States: **ES GR**

(71) Applicant: **SCHERING CORPORATION**
**2000 Galloping Hill Road**
**Kenilworth New Jersey 07033(US)**

(72) Inventor: **Vogel, Ernst**
**Museggstrasse**
**CH-6017 Ruediswil(CH)**

(74) Representative: **von Kreisler, Alek, Dipl.-Chem. et al**
**Patentanwälte Von Kreisler-Selting-Werner**
**Deichmannhaus am Hauptbahnhof**
**D-5000 Köln 1(DE)**

(54) **Processes and compounds useful for resolving 1-methyl-3-phenylpropylamine.**

(57) Processes and intermediates for resolving 1-methyl-3-phenylpropylamine are disclosed.

EP 0 320 898 A2

# PROCESSES AND COMPOUNDS USEFUL FOR RESOLVING 1-METHYL-3-PHENYLPROPYLAMINE

## BACKGROUND OF THE INVENTION

Dilevalol, 5-[(R)-1-hydroxy-2-[(R)-(1-methyl-3-phenylpropyl)amino]ethyl]salicylamide, is a recently developed antihypertensive agent. It is a compound having two asymmetric centers and thus its chemical synthesis is complex.

Various processes have been described for preparing dilevalol. See for example: Gold et al. U.S. Patent No. 4,658,060; Gold et al. U.S. Patent No. 4,619,919; and Gold et al., J. Med. Chem. 1982, 25, 1363-1370. It would be highly desirable if dilevalol could be prepared economically and in high purity from its enantiomeric precursors.

One precursor proposed for the synthesis of dilevalol is (R)-1-methyl-3-phenylpropylamine ((R)-MPPA), since this compound contains one of the two chiral centers of the dilevalol molecule. The compound must, however, be available as a single enantiomer with high enantiomeric purity. Since racemic 1-methyl-3-phenylpropylamine is commercially available on a large scale, it would also be highly desirable to have a simple resolution process which would efficiently provide (R)-MPPA in such purity from the racemate.

One method used for resolving amines is the crystallization of the racemic amine with an optically active acid to form two diastereomeric salts, one of which is relatively insoluble. Two resolution procedures of this type are described in the literature in connection with 1-methyl-3-phenylpropylamine. In the first procedure, van Dijk et al. in Recl. Trav. Chim. Pays-Bas, 82, 189 (1963), propose to allow racemic 1-methyl-3-phenylpropylamine to crystallize as its D(-) mandelic acid salt using ethanol as the solvent. After several recrystallizations, pure (S)-1-methyl-3-phenylpropylamine D-mandelate salt is obtained, and impure (R)-1-methyl-3-phenylpropylamine D-mandelate is obtained in low yield from the mother liquors. Thus, it is the (S)-MPPA that is selectively precipitated and isolatable in pure form, whereas the (R)-MPPA remains in the mother liquor and is much more difficult to purify.

The second procedure, described by Cervinka et al. in Coll. Czech. Chem. Commun., 33, 3551 (1968), uses L-tartaric acid in the resolution of 1-methyl-3-phenylpropylamine. After one crystallization of the amine hydrogen tartrate from ethanol, they claimed to have isolated enantiomerically impure (R)-MPPA amine after liberation. However, when this procedure was repeated by the Applicant, pure (S)-MPPA hydrogen tartrate (not (R)) was isolated after three recrystallizations.

Both literature procedures therefore have the significant drawback of producing the undesired S-isomer as the crystalline salt. Purification of (R)-MPPA salt from the mother liquor to higher than 95% pure (R)-MPPA is a lengthy and low-yielding procedure, which is not well suited for a large-scale production. Although the problem might be solved by using unnatural D-tartaric acid as a resolving acid, this acid is expensive, especially on a large scale, and recovery is difficult.

## SUMMARY OF THE INVENTION

It has now unexpectedly been found that high yields of high purity (R)-1-methyl-3-phenylpropylamine can be prepared from a mixture containing said compound along with (S)-1-methyl-3-phenylpropylamine by contacting a solution of said compounds or of their soluble salts with an effective amount of a compound of the formula I

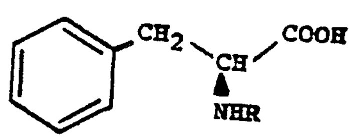

I

to form a salt of the formula II

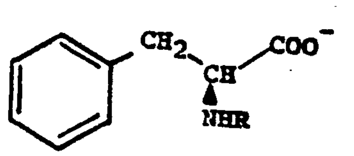 • 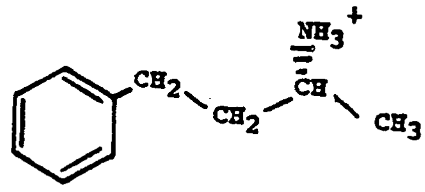

II

which selectively precipitates from said solution; wherein R is $-COR^1$, $-CONR^2R^3$ or $-SO_2R^4$; $R^1$ is H, alkyl, substituted alkyl, alkoxy, substituted alkoxy, allyloxy, phenyl, substituted phenyl, phenyloxy, substituted phenyloxy, benzyl, substituted benzyl, benzyloxy, substituted benzyloxy or 2-furanylmethoxy; $R^2$ and $R^3$ may be the same or different and each is independently selected from H, alkyl, substituted alkyl, phenyl, substituted phenyl, benzyl or substituted benzyl; and $R^4$ is alkyl, substituted alkyl, phenyl, substituted phenyl, benzyl or substituted benzyl. This process provides a number of distinct advantages. It may employ common polar solvents such as lower alkyl alcohols, e.g. ethanol, lower alkyl ketones, e.g., acetone, and/or water. The (S)-MPPA, which is separated in the mother liquor, is readily racemized and thus can be recycled for further production of (R)-MPPA. Moreover, the compound of formula I is easily cleaved from the salt of formula II and can also be recycled for further use in the production of (R)-MPPA. Overall, an efficient, economic process is achieved for producing high purity (R)-MPPA which can then likewise be used to prepare high purity dilevalol.

In a preferred embodiment of the invention the process is performed with a compound of the formula Ia or Ib:

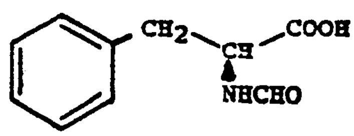

Ia or

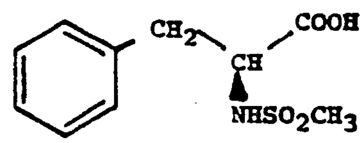

Ib.

N-formyl-L-phenylalanine is particularly preferred. This compound is easily prepared; it produces an N-formyl-L-phenylalanine/(R)-MPPA salt with high enantiomeric purity in two recrystallizations and in high yield above 70% based on available (R)-MPPA; water may be used as the recrystallization solvent and this is economically very advantageous; and N-formyl-L-phenylalanine is easily recovered in over 90% yield by alkaline cleavage of the salt, extraction of the (R)-MPPA from the alkaline phase and acidification of the alkaline phase. N-formyl-L-phenylalanine is chemically and optically stable in solutions at a pH of from 2 to 12.

The invention also involves compounds of formula II

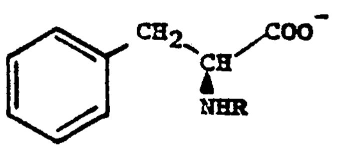 • 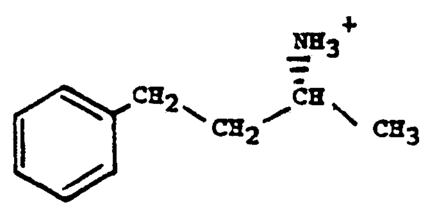

II

wherein R is as defined above. The preferred compounds of formula II are:

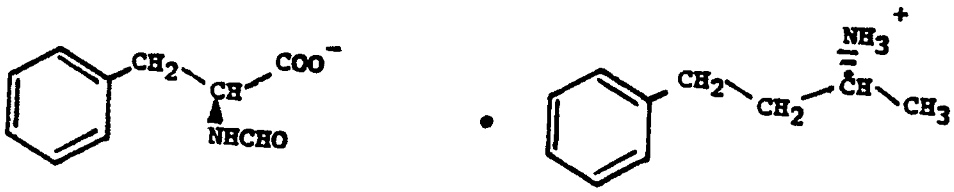

and

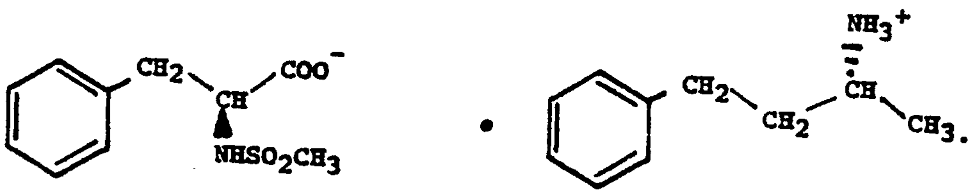

## DETAILED DESCRIPTION OF THE INVENTION

Unless otherwise indicated, the following terms have the following meanings:

alkyl (including the alkyl portions of substituted alkyl, alkoxy, substituted alkoxy, alkanol, alkyl ketone, etc.) - a straight or branched carbon chain containing from 1 to 6 carbon atoms;

substituted alkyl (including the substituted alkyl portion of substituted alkoxy) - an alkyl group in which at least one H is replaced by a fluoro, chloro or bromo; and

substituted phenyl and substituted benzyl (including the substituted phenyl and substituted benzyl portions of substituted phenyloxy and substituted benzyloxy) - a phenyl or benzyl group on which at least one H of the phenyl ring is replaced by fluoro, chloro, bromo, nitro, alkyl or alkoxy.

Suitable COR[1] groups include CHO, $COCH_3$, $COCH_2CH_3$, $COCH_2CH_2CH_3$, $COCH_2Cl$, $COCHCl_2$, $COCCl_3$, $COCF_3$, COPh (Ph = phenyl), CO-(p-$NO_2$Ph), (CO)$OCH_3$, (CO)$OCH_2CH_3$, (CO)$OCH_2CH_2CH_3$, (CO)-$OCH_2CH_2X$, (CO)$OCH_2CHX_2$, (CO)$OCH_2CX_3$, (CO)O-allyl, (CO)O-benzyl, (CO)O-t-butyl, (CO)O(p-methoxybenzyl), (CO)O(p-nitrobenzyl), (CO)O(o-, m- or p-X-benzyl) (wherein X is fluoro, chloro or bromo), and CO)-O(2-furanylmethyl). Suitable $CONR^2R^3$ groups include $CONH_2$, $CONHCH_3$, $CON(CH_3)_2$, $CON(CH_2CH_3)_2$ and CONH(t-butyl). Suitable $SO_2R^4$ groups include $SO_2CH_3$, $SO_2CH_2CH_3$, $SO_2CH_2CH_2CH_3$, $SO_2$Ph and $SO_2$(p-tolyl).

The compounds of formula I either are known materials or may be prepared by methods well-known in the art. For example, the compounds of formula I wherein R is R[1] may be made by well-known acylation methods.

In the process of the invention, a solution containing a mixture, usually a racemate, of (R)- and (S)-MPPA is contacted with an effective amount of a compound of formula I

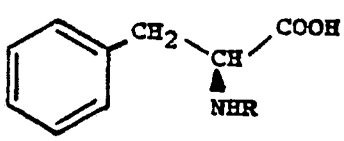

I

to form a selectively insoluble salt of formula II, wherein R is as defined above. The solvent employed may be any conventional polar solvent which dissolves the mixture of (R) and (S) amines and preferably also dissolves the compound of formula I. Also, the salt of formula II is selectively insoluble in the solvent with respect to the corresponding (S)-MPPA salt. In theory, the compound of formula I could be employed as a slurry or suspension in contacting the solution, so long as the compound of formula II is less soluble than the compound of formula I and sufficient time is allowed for equilibrium to be obtained. Suitable solvents include, for example, lower ($C_1$-$C_6$) alkanols such as methanol, ethanol, propanol, isopropanol, etc.; lower alkyl ($C_3$-$C_6$) ketones such as acetone, methyl ethyl ketone, etc.; dioxane; glyme (1,2-dimethoxyethane) and similar materials; water; or one or more combinations of such solvents. Preferred solvents include lower

alcohols especially ethanol, acetone, water or combinations thereof. With N-formyl-L-phenylalanine as the compound of formula I, water is the preferred solvent since it works very efficiently and effectively and is most economical with fewest disposal problems.

In forming the relatively insoluble salt of formula II, the treated solution may be heated, to achieve dissolution of the amine mixture and compound I, if desired. The treated solution is normally cooled to a temperature effective to achieve selective separation of solid compound of formula II, usually to maximize separation and yield. Preferably, the solution is cooled slowly to increase the resolution of the amine salts. The final temperature selected will depend on the compound of formula I and solvent system employed.

The compound of formula I is employed in an amount effective to produce the desired solid form of the compound of formula II so that it may be selectively precipitated from its corresponding (S)-MPPA salt. Preferably, from about 0.3 to about 1.5 equivalents, more preferably from about 0.5 to about 1.0 equivalents, of the compound of formula I is employed. However, more or less compound of formula I may be employed, if desired. Other acids, including organic acids, e.g., acetic acid, or inorganic acids, may also be employed in the process of the invention to neutralize the remaining amines in the mixture, e.g., the (S)-MPPA, with such neutralized amine being soluble and thus separable from the precipitated compound of formula II.

The separation of the compound of formula II may be accomplished by any suitable means for separating solids from liquids, e.g., filtration, decantation, centrifugation, etc. Filtration is preferred.

The material separated from the mother liquor may be recrystallized by conventional means in the same or similar solvent system. For example, in one embodiment of the invention, a mixture of 1 equivalent of racemic 1-methyl-3-phenylpropylamine with 0.5 to 1 equivalent of N-formyl-L-phenylalanine in 1 to 12 parts water, preferably 3 to 4 parts water, may be heated to 50° to 60° C until a solution is obtained. If only 0.5 equivalent N-formyl-L-phenylalanine is used, 0.5 equivalent of an inorganic or organic acid such as hydrochloric, sulfuric, formic or acetic acid is preferably added. The solution is cooled slowly and stirred at 20° C for 1 to 24 hours. After filtration and washing, the salt of formula II is slurried in 3 to 10 parts water, preferably 3 to 4 parts water, and heated to 50° to 60° C until a solution is obtained. The solution is cooled to 20° C and stirred for 2 to 24 hours to provide a purified compound of formula II in about 70% yield.

After the desired number of recrystallizations, the salt of formula II may be cleaved by reaction with strong acid or strong base, preferably strong base, by techniques well-known in the art. Not more than one equivalent of base should preferably be used. Suitable acids and bases include aqueous HCl, NaOH, KOH solutions, etc. For example, an N-formyl-L-phenylalanine salt of formula II may be slurried with 4 parts water and the pH adjusted to 12-13 with NaOH (one equivalent) to cleave the salt and form the free (R)-MPPA.

The free (R)-MPPA may be treated by conventional means for separation. For example, the free (R)-MPPA may be extracted with a suitable solvent, e.g., a water-immiscible solvent, such as methylene chloride, ethyl acetate, diethyl ether, t-butyl methylether, and in particular toluene. The extract may then be evaporated and the (R)-MPPA distilled to give high purity R-MPPA, preferably in over 95% enantiomeric purity.

The mother liquors from the crystallizations contain (S)-MPPA in salt form. Such (S)-MPPA can be separated from the mother liquor and subjected to racemization, e.g., by the methods described in German Offenlegungsschrift 2 903 589. One embodiment involves heating the (S)-MPPA with Raney nickel in an $H_2$ atmosphere. Preferably the $H_2$ pressure is from about 10 to about 500 bar and the temperature is from about 50 to about 300° C. The racemization is preferably carried out in the substantial absence of solvent. The racemized material may then be employed (recycled) as a starting material in the process of the invention.

For example, the mother liquors containing (S)-MPPA may be collected and their pH adjusted to pH 12-13. The free (S)-MPPA thus formed may be extracted with any suitable organic solvent, e.g., methylene chloride, but preferably toluene. After evaporation of the solvent, enriched (S)-MPPA is obtained. This may be heated with 0.02 to 0.1 part water and wet Raney nickel in an autoclave for 10 to 20 hours at 100° to 200° C and at a pressure of 1034 kilo-pascals hydrogen (150 psi hydrogen) for a suitable time, usually for about 16 to 20 hours. Relatively pure racemic 1-methyl-3-phenylpropylamine is obtained after filtration of the catalyst and distillation of the amine.

The resolving compound of formula I is also easily recovered for further use. In one embodiment, after the (R)-MPPA and (S)-MPPA have been extracted as described above, the remaining liquid phases may be collected and concentrated by conventional means. The pH may be adjusted to 2 with strong acid such as HCl. The compound of formula II, e.g., N-formyl-L-phenylalanine, may then be separated by, for example, filtration for further use in the process of the invention.

The following examples are intended to illustrate, but not to limit, the present invention.

## Example 1

a) Salt formation:

To a suspension of 1.932 kg (10 moles) N-formyl-L-phenylalanine in 12.66 liters water, 1.492 kg (10 moles) (R,S)-1-methyl-3-phenylpropylamine was added at once. The reaction mixture was heated to 60 ° C, and the clear solution was cooled slowly until crystallization started. The suspension was cooled further to 20 ° C and stirred for 24 hours at 17-22 ° C. The product was filtered off and washed with 950 ml cold water. Yield: 2.485 kg of R-MPPA/N-formyl-L-phenylalanine salt (III), water-wet.

The mother liquor was concentrated to about 9 liters volume and by the salt cleavage procedure described below 741.7 g (49.7%) enriched (S)-MPPA and 853 g (44.1%) N-formyl-L-phenylalanine were recovered.

b) Recrystallization:

The water-wet salt III (2.485 kg) from step (a) above was recrystallized from 5.1 liters of water by heating to 60 ° C and slow cooling to 20 ° C. After stirring for 20 hours at 20 ° C, the product was filtered off, washed with 750 ml cold water and dried in a vacuum oven at 60 ° C. Yield: 1.34 kg (39% based on (R,S)-amine) white R-MPPA/N-formyl-L-phenylalanine salt (III), m.p. 138-139 ° C.

The mother liquor was concentrated to about 1.5 liters volume and 147.3 g (10%) enriched (S)-MPPA and 200.5 g (10.3%) N-formyl-L-phenylalanine were recovered.

c) Salt cleavage:

1.33 kg of the salt III was suspended in 5.16 liters of water and cooled to 5 to 10 ° C, and the pH was adjusted to 12.5 - 13.0 with 30% sodium hydroxide solution. The resulting solution was extracted 4 times with a total of 6 liters of methylene chloride. After washing the combined organic phases with 3 liters of water, the solvent was evaporated *in vacuo*, and the oily residue was distilled at 50 ° C/0.7 mbar.

Yield: 550 g (R)-MPPA
(95% in salt cleavage; 36.8% overall)
$[\alpha]_D^{20}$ : -18.0 ° (c = 5 in cyclohexane)
enantiomeric purity: 97.3% (R)-MPPA

d) Salt cleavage:

1.00 kg of the salt III was suspended in 1.15 liters of water and cooled to 10 to 15 ° C, and the pH was adjusted to 12.5 - 13.0 with 30% sodium hydroxide solution (one equivalent). The resulting solution was extracted 4 times with a total of 2.4 liters of toluene at 25-30 ° C. The combined organic phases were washed with 1 liter of water, the solvent was evaporated *in vacuo*, and the oily residue was distilled at 50 ° C/0.7 mbar.

Yield: 406 g (R)-MPPA
(93% in salt cleavage; 36.3% overall)
$[\alpha]_D^{20}$ : -18.0 ° (c = 5 in cyclohexane)
enantiomeric purity: 97.3% (R)-MPPA

e) N-Formyl-L-phenylalanine recovery:

The aqueous layer from step (c) (or (d)) above after amine extraction was adjusted to pH 2.0 with concentrated hydrochloric acid. The suspension was stirred at 10 ° C for 2 hours, and the product was filtered off, washed with 7 liters cold water and dried *in vacuo* at 60 ° C. Yield: 685 g (35.5%) N-formyl-L-phenylalanine.

m.p.: 167° C
enantiomeric purity: 99.9%L
total recovery of N-formyl-L-phenylalanine: 1738.5 g (90%).

## Example 2

a) Salt formation:

To a suspension of 97.8 g (0.4 mole) N-methanesulfonyl-L-phenylalanine in 1.1 liter acetone, 60 g (0.4 mole) of (R,S)-1-methyl-3-phenylpropylamine was added. The reaction mixture was heated to reflux until a clear solution was obtained, and then cooled to 20° C. After stirring for 24 hours, the product was filtered off, washed, and dried in vacuo.

Yield: 75 g (47.5%) of (R)-MPPA/N-methanesulfonyl-L-phenylalanine salt (IV).

b) Recrystallization:

74 g of the enriched salt IV was recrystallized from 770 ml of hot acetone. After cooling to 20° C and stirring for 24 hours, the product was filtered off, washed, and dried in vacuo.

Yield: 50.1 g (67.7% in recryst.) of salt IV
melting point: 171.5 - 172° C
optical rotation: $[\alpha]_D^{20}$ = 2.9° (c = 5 in $CH_3OH$)
enantiomeric purity (amine): 95%R

The free (R)-MPPA can be obtained from the salt IV by the same procedure as described in Example 1 above.

## Example 3

a) Salt formation:

To a suspension of 96.6 g (0.5 mole) N-formyl-L-phenylalanine in 606 ml water, 30.1 g (0.5 mole) acetic acid and 149.2 g (1 mole) (R,S)-1-methyl-3-phenylpropylamine were added. The reaction mixture was heated to 60° C and the resulting clear solution was cooled slowly until crystallization started. The suspension was cooled further to 3° C and stirred for 24 hours at 0° to 5° C. The product was filtered off and washed twice with 45 ml cold water to yield after drying at 60° C: 150.0 g of R-MPPA/N-formyl-L-phenylalanine salt (V).

b) Recrystallization

The salt V (146.3 g) from step a) above was recrystallized from 438 ml water by heating to 60° C and slow cooling to 3° C. After stirring for 24 hours at 0° to 5° C, the product was filtered off, washed twice with 65 ml cold water, and dried in a vacuum oven at 60° C to yield: 137.2 g (41% based on (R,S)-MPPA) white R-MPPA/N-formyl-L-phenylalanine salt (V), m.p. 136°-137° C; enantiomeric purity (MPPA): 96.8%R.

The (S)-MPPA recovery, salt cleavage and N-formyl-L-phenylalanine recovery may be performed as in Example 1 above.

By essentially the same crystallization procedures as described above, employing the amounts of racemic amine (R,S-MPPA), the resolving acids, and solvents as indicated in the first three columns of

Table 1 below, the results (yield and enantiomeric purity as % (R)-MPPA) as listed in the four right hand columns of Table 1 were obtained:

TABLE 1

| Amount of Racemic Amine (moles) | Resolving acid/(equiv.) | Solvent/concentration of salt | First Recrystallization | | Second Recrystallization | |
|---|---|---|---|---|---|---|
| | | | Yield | %R | Yield | %R |
| 0.10 | N-mesyl-L-Phe[1] (1 eq.) | methylene chloride/50%[2]<br>methylene chloride/20%[3] | 52% | 72% | 76% | 86% |
| 0.10 | N-mesyl-L-Phe[1] (1 eq.) | dioxan/8.3%[2]<br>dioxan/12.5%[3] | 59% | 77% | 79% | 90% |
| 0.40 | N-formyl-L-Phe[1] (1 eq.) | methanol + water (1:5)/0.04%[4] | 31% | 90% | 67% | 97.4% |
| 0.40 | N-formyl-L-Phe[1] (1 eq.) | water/12.5%[4] | 45% | 85% | 66% | 97.0% |
| 0.40 | N-formyl-L-Phe[1] (1 eq.) | water/33%[2]<br>water/27%[3] | ca. 50% | 85% | 78% | 97.3% |
| 1.0 | N-formyl-L-Phe (0.5 eq.) + acetic acid (0.5 eq.) | water/45.5%[2]<br>water/33.3%[3] | 43.8% | 87.3% | 93.8% | 96.8% |

[1] Phe = phenylalanine
[2] First recrystallization
[3] Second recrystallization
[4] Same solvent system for both recrystallizations

Whereas the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. A process for the resolution of a mixture of (R)-1-methyl-3-phenylpropylamine and (S)-1-methyl-3-phenylpropylamine, which comprises contacting a solution containing said mixture or soluble salts thereof with an effective amount of a compound of the formula I

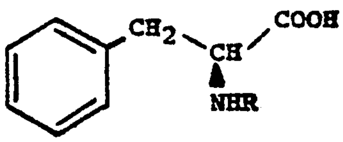

I

to form a salt of the formula II

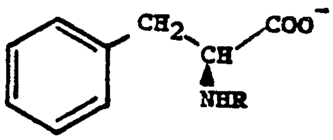

•

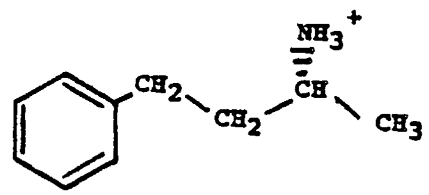

II (R)

which selectively precipitates from said solution, wherein R is $-COR^1$, $-CONR^2R^3$ or $-SO_2R^4$; $R^1$ is H, alkyl, substituted alkyl, alkoxy, substituted alkoxy, allyloxy, phenyl, substituted phenyl, phenyloxy, substituted phenyloxy, benzyl, substituted benzyl, benzyloxy, substituted benzyloxy or 2-furanylmethoxy; $R^2$ and $R^3$ may be the same or different and each is independently selected from H, alkyl, substituted alkyl, phenyl, substituted phenyl, benzyl and substituted benzyl; and $R^4$ is alkyl, substituted alkyl, phenyl, substituted phenyl, benzyl or substituted benzyl.

2. A process according to claim 1, wherein the solution comprises water, lower alkanols, lower alkyl ketones or mixtures thereof as solvent.

3. A process according to claim 2, wherein the solution comprises water, ethanol, acetone or mixtures thereof as solvent.

4. A process according to claim 1, wherein the amount of the compound of formula I added to the solution is from about 0.3 to about 1.5 equivalents relative to (R)-1-methyl-3-phenylpropylamine.

5. A process according to claim 1, wherein the amount of the compound of formula I added to the solution is from about 0.5 to about 1.0 equivalent relative to (R)-1-methyl-3-phenylpropylamine.

6. A process according to claims 2, 3 or 4, wherein the compound of formula I is

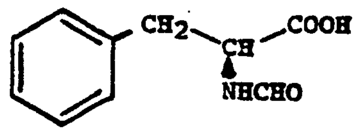

Ia

or

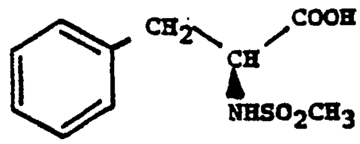

Ib.

7. A process according to claim 6, wherein the solution is a aqueous solution and the compound of formula I is

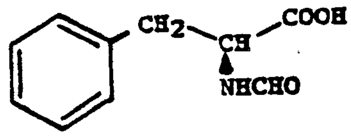

Ia.

8. A process according to claim 1, wherein the contacted solution is cooled to precipitate the salt of formula II.

9. A process according to claim 1, further comprising separating the precipitated salt of formula II from the remaining solution.

10. A process according to claim 9, further comprising treating the separated salt of formula II with acid or base to cleave the salt and produce a mixture containing free (R)-1-methyl-3-phenylpropylamine or its acid addition salt and a compound of formula I.

11. A process as claimed in claim 10, wherein up to one equivalent of base is used to cleave the salt.

12. A process according to claim 10 or claim 11, further comprising contacting said mixture containing free (R)-1-methyl-3-phenylpropylamine with an immiscible solvent which extracts free (R)-1-methyl-3-phenylpropylamine to form (1) an extract solution containing extracted (R)-1-methyl-3-phenylpropylamine and (2) a residual solution.

13. A process according tc claim 12, further comprising acidifying and cooling said residual solution to precipitate the compound of formula I, which precipitate is then separated from said residual solution.

14. A process according to claim 1, wherein the compound of formula I is N-formyl-L-phenylalanine.

15. A process according to claim 9, further comprising racemizing the (S)-1-methyl-3-phenylpropylamine in the remaining solution.

16. A process according to claim 15, further comprising recyclizing the racemized material for use in said contacting with said compound of formula I.

17. A compound of the formula II

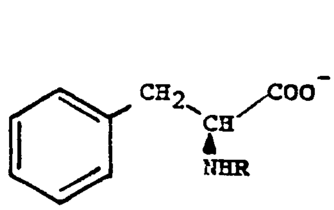

•

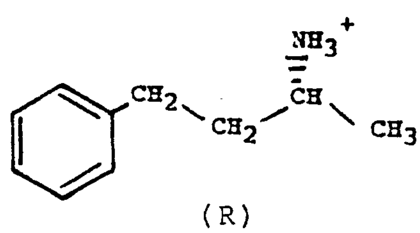

(R)

II

wherein R is $-COR^1$, $-CONR^2R^3$ or $-SO_2R^4$; $R^1$ is H, alkyl, substituted alkyl, alkoxy, substituted alkoxy, allyloxy, phenyl, substituted phenyl, phenyloxy, substituted phenyloxy, benzyl, substituted benzyl, benzyloxy, substituted benzyloxy or 2-furanylmethoxy; $R^2$ and $R^3$ may be the same or different and each is independently selected from H, alkyl, substituted alkyl, phenyl, substituted phenyl, benzyl and substituted benzyl; and $R^4$ is alkyl, substituted alkyl, phenyl, substituted phenyl, benzyl or substituted benzyl.

18. A compound according to claim 17, wherein R is CHO, COO-benzyl or $SO_2CH_3$.

19. A compound according to claim 17, which is

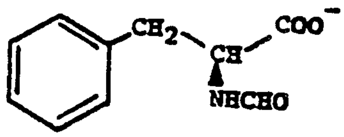

•

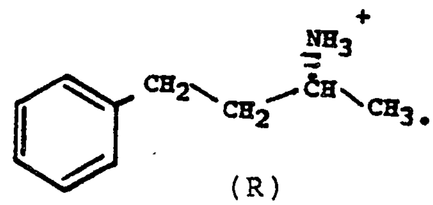

(R)

20. A compound according to claim 17, which is

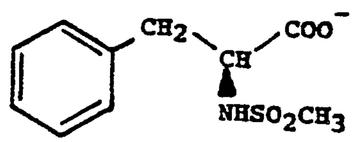

•

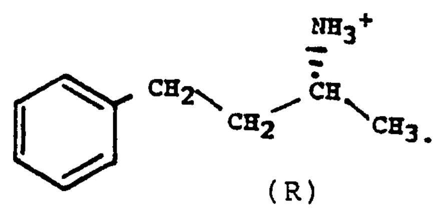

(R)